# EUROPÄISCHE PATENTSCHRIFT

(11) **EP 1 434 523 B1**
(45) Veröffentlichungstag und Bekanntmachung des Hinweises auf die Patenterteilung: **25.07.2007**
(21) Anmeldenummer: 02776698.9
(22) Anmeldetag: 24.09.2002
(51) Int. Cl.: A61B 17/00

(54) **MULTIFUNKTIONSINSTRUMENT FÜR DIE MIKROINVASIVE CHIRURGIE**
MULTIFUNCTIONAL INSTRUMENT FOR USE IN MICROINVASIVE SURGERY
INSTRUMENT MULTIFONCTIONS POUR CHIRURGIE MICRO-INVASIVE

(30) Priorität: 25.09.2001 DE 10147145
(43) Veröffentlichungstag der Anmeldung: 07.07.2004
(73) Patentinhaber: Kunz, Reiner, 14532 Kleinmachnow (DE)
(72) Erfinder: Kunz, Reiner, 14532 Kleinmachnow (DE)
(74) Vertreter: König, Beate
(86) Internationale Anmeldenummer: PCT/DE2002/003581
(87) Internationale Veröffentlichungsnummer: WO 2003/026511

(56) Entgegenhaltungen:
- EP-A- 0 692 224
- US-A- 5 350 355

## Beschreibung

Die Erfindung betrifft ein Multifunktionsinstrument für die mikroinvasive Chirurgie mit einem Bedienungshandgriff, einem am Bedienungshandgriff befestigten mehrlumigen Rohr, mindestens zwei im Rohr koaxial ausgebildeten Führungskanälen, sowie in den Führungskanälen verschieblich und drehbar gelagerten chirurgischen Instrumenten mit einem Schaft, an dessen distalem Ende jeweils ein chirurgisches Arbeitselement angeordnet ist, wobei die Instrumente zwischen einer Ruheposition, in der das jeweilige Arbeitselement in das Rohr zurückgezogen ist, und einer Arbeitsposition, in der das jeweilige Arbeitselement aus dem distalen Ende des Rohrs herausragt, verschieblich ist.

In operativ tätigen medizinischen Bereichen (Chirurgie, Gynäkologie, Urologie, Neurochirurgie etc.) wird ständig versucht, das operative Trauma, d.h. die Schädigung des Patienten durch die Operation selbst zu verringern. Eine Möglichkeit zur Reduktion des operativen Traumas besteht in der Minimalisierung des operativen Zugangswegs.

Vor über einem Jahrzehnt wurde begonnen, operative Eingriffe im Bauchraum mittels einer Laparoskopie durchzuführen. Hierbei werden neben einer Optik, gekoppelt mit einer Videokette, über mehrere gesonderte Zugangswege verschiedene Einzelinstrumente in die Bauchhöhle eingeführt, die zur Durchführung der Operation erforderlich sind. Der eigentliche Eingriff im Bauchraum wird entsprechend einer Operation mit klassischem Hautschnitt vom Prinzip her unverändert vorgenommen, so daß als prinzipieller Unterschied der klassische operative Zugang mittels Durchtrennung der Bauchdecke wegfällt.

Bei minimal-invasivem Zugangsweg fallen die Streßreaktionen des Organismus während und nach der OP wesentlich geringer aus. Als Resultat ist der operative Eingriff schmerzärmer, der Patient ist schneller mobilisierbar, eher entlassungsfähig und kann seine Aktivitäten und Berufstätigkeit schneller wieder aufnehmen.

Die auf dem Markt befindlichen laparoskopischen Operationsinstrumente kombinieren in der Regel maximal 2 Funktionen. Das bedeutet, daß der typische chirurgische Vorgang, nämlich Freilegen einer Struktur, Verschluß (z.B. mit Clips), Durchschneiden der Struktur, Freilegen weiterer Strukturen mit dem mehrfachen Wechsel von Operationsinstrumenten verbunden ist. Messungen haben ergeben, daß ein Instrumentenwechsel bei minimal invasiven Eingriffen von der Entscheidung zum Instrumentenwechsel bis zum Positionieren des neuen Instruments im Operationsgebiet ca. 40 Sekunden benötigt. Eine Verkürzung dieses Zeitintervalls kann zu einer erheblichen Verkürzung der Gesamt-Operationsdauer führen. Werden die verschiedenen notwendigen Instrumentenfunktionen in einem Gerät vorgehalten, entfällt ggf. sogar die Teilnahme einer Operationsschwester oder eines Operationsassistenten über weite Teile der Operation.

Aus der EP 0 692 224 B1 ist ein Multifunktionsinstrument für die mikroinvasive Chirurgie der Eingangs genannten Gattung bekannt. Eines der Probleme bei dem dort gezeigten Multifunktionsinstruments besteht darin, daß die Auswahl der chirurgischen Instrumente sowie die Drehung des Rohres unter Zuhilfenahme von manueller Wirkenergie erfolgen muss. Hierdurch kann es zum Verreißen des Multifunktionsinstruments beim Instrumentenwechsel kommen. Außerdem wird für diesen Wechselvorgang häufig auch die zweite Hand benötigt. Das Zurückziehen, Auswählen und Wiedervorschieben des ausgewählten chirurgischen Instruments muß ebenfalls vom Operateur sensorisch gesteuert werden.

Ein weiteres Problem des bekannten Multifunktionsinstruments liegt darin, daß bei der Zurückführung des jeweils verwendeten chirurgischen Instruments mittels Federkraft eine Reaktionskraft bzw. ein Reaktionsimpuls auf das Multifunktionsinstrument ausgeübt wird. Zwar kann dieser unerwünschte Reaktionsimpuls durch entsprechende Dämpfungsglieder verringert werden, solche Dämpfungsglieder bedeuten jedoch andererseits zusätzliches Gewicht des Multifunktionsinstruments, welches zum Zwecke der guten Handhabbarkeit grundsätzlich so leicht wie möglich gehalten werden sollte. Ergänzend sei angemerkt, daß hier für das Verbringen der chirurgischen Instrumente in die Arbeitsstellung, bei der die genannte Feder gespannt wird, ebenfalls manuelle Wirkenergie seitens des Operateurs eingebracht werden muß.

Der vorliegenden Erfindung liegt im wesentlichen die Aufgabe zugrunde, ein Multifunktionsinstrument für die mikroinvasive Chirurgie zu schaffen, welches einen einfachen und schnellen Instrumentenwechsel ermöglicht und hierbei den Operateur entlastet, wobei gleichzeitig unerwünschte Nebenwirkungen wie Verreißen des Multifunktionsinstruments und Reaktionsimpulse vermieden werden sollen.

Diese Aufgabe wird erfindungsgemäß im wesentlichen dadurch gelöst, daß gemäß dem Kennzeichen des Patentanspruchs 1 die Instrumente in die und aus der Arbeitsposition motorisch verfahrbar sind, daß wenigstens eines der Instrumente in seiner Arbeitsposition motorisch drehbar ist und daß eine elektronische Steuerung zum Steuern zumindest der motorischen Verfahrbewegungen und Drehbewegungen vorgesehen ist.

In bevorzugter Weiterbildung der Erfindung ist vorgesehen, daß das mehrlumige Rohr am Bedienungshandgriff drehbar gelagert ist und ebenfalls motorisch drehbar ist, wobei die Drehbewegung mittels der elektronischen Steuerung steuerbar ist.

Aufgrund des motorischen Aus- und Einfahrens der chirurgischen Instrumente sowie des motorischen Drehens des mehrlumigen Rohrs zur Auswahl des jeweils gewünschten chirurgischen Instruments wird der Operateur von den entsprechenden manuellen Bewegungsabläufen entlastet, ein Verreißen des Multifunktionsinstruments beim Instrumentenwechsel ist ausgeschlossen und Reaktionsimpulse aufgrund Rückstellfedern können durch eine geeignete Ansteuerung der Motoren praktisch vollständig vermieden werden. Darüberhinaus ermöglich das erfindungsgemäße Multifunktionsinstrument einen echten Einhandbetrieb.

Die Befehlseingabe für die elektronische Steuerung kann über am Bedienhandgriff angeordnete Betätigungselemente wie beispielsweise Tasten erfolgen. In alternativerweise kann eine sprachliche Befehlseingabe mit einer entsprechenden Sprachsteuerung vorgesehen sein. Die von der Steuerung zu verarbeitenden Befehle sollten in diesem Fall zumindest das Einnehmen der Ruhe- oder Arbeitspositionen der chirugischen Instrumente oder das Drehen der chirurgischen Instrumente umfassen. Vorteilhaft kann auch sein, wenn die Befehlseingabe wahlweise über Sprache oder Betätigungselemente erfolgt.

Zum motorischen Antrieb kommen vorzugsweise Elektromotoren, beispielsweise Mikrogetriebemotoren, zum Einsatz, die im Bedienungshandgriff des Multifunktionsinstruments angeordnet sind. In bevorzugter Weiterbildung der Erfindung ist vorgesehen, daß die den chirurgischen Instrumenten zugeordneten Elektromotoren über zwischen das jeweilige Instrument und den zugeordneten Elektromotor geschaltete Kopplungselemente selektiv mit dem jeweils auszuwählenden chirurgischen Instrument koppelbar sind, wobei diese selektive Kopplung vorzugsweise durch eine Relativbewegung zwischen Instrument und zugeordnetem Elektromotor bzw. zugeordnetem Kopplungselement erfolgt. Eine besonders einfache Konstruktion ergibt sich, wenn die Elektromotoren im Handgriff ortsfest angeordnet sind und die selektive Kopplung im wesentlichen durch eine geeignete Rotation des Rohrs erfolgt, wobei je nach Winkelstellung des Rohrs unterschiedliche chirurgische Instrumente mit den jeweils zugeordneten Elektromotoren für eine Axialbewegung bzw. eine Rotationsbewegung gekoppelt sind.

Die Kraftübertragung von den Antriebsmotoren zu den chirurgischen Instrumenten erfolgt vorzugsweise über geeignete Zahnräder wie Ritzel, Schneckenräder, usw. Zur Aufnahme der Antriebskraft weist gemäß einem weiteren vorteilhaften Merkmal der Erfindung ein proximaler Endabschnitt des Schafts eines jeden Instruments, der im in das Rohr eingesetzten Zustand aus dem proximalen Ende des Rohrs heraussteht, geeignete Zahnungen auf. Im Einzelnen weist der Schaft zumindest eines der chirurgischen Instrumente, vorzugsweise aller chirurgischen Instrumente, einen ersten Zahnabschnitt für das Verschieben des Instruments in axialer Richtung und einen zweiten Zahnabschnitt für das Drehen des chirurgischen Instruments auf.

Gemäß einer vorteilhaften Weiterbildung der Erfindung weist der Schaft zumindest eines der chirugischen Instrumente an seinem proximalen Ende ein Kupplungselement zum Eingriff mit einem Betätigungselement zum manuellen Betätigen des Instruments auf. Sobald das jeweils ausgewählte chirurgische Instrument über das Kupplungselement mit dem Betätigungselement gekuppelt ist und mittels des zugeordneten Elektromotors in seine Arbeitsstellung verfahren wurde, kann es nunmehr vom Operateur manuell betätigt werden; ein solches manuelles Betätigen ist für chirurgische Instrumente, bei denen eine taktile Rückmeldung notwendig oder wünschenswert ist, beispielsweise bei Scheren oder Fassapplikatoren, vorgesehen, während beispielsweise bei Clipapplikatoren eine taktile Rückmeldung nicht unbedingt erforderlich ist und damit auch ein motorischer Arbeitshub vorgesehen sein kann.

Demgemäß ist in weiterer vorteilhafter Ausbildung der Erfindung vorgesehen, daß mindestens zwei der chirurgischen Instrumente, z. B. Schere und Fassapplikator, einen kurzen Arbeitshub bei taktiler Rückmeldung aufweisen, und, nach motorischem Verbringen in ihre Arbeitsposition, manuell betätigbar sind, und daß mindestens eines der chirurgischen Instrumente, z. B. Clipapplikator mit langem Arbeitshub, nach motorischem Verbringen in seine Arbeitsposition manuell oder motorisch betätigbar ist.

Um eine möglichst große Anwendungsbreite des erfindungsgemäßen Multifunktionsinstruments zu ermöglichen, weist das Rohr vorzugsweise Führungskanäle unterschiedlichen Durchmessers auf, nachdem beispielsweise Clipapplikatoren einen größeren Durchmesser benötigen als Scheren. Vorzugsweise weist das Rohr mindestens drei Führungskanäle auf, insbesondere zwei Führungskanäle mit einem ersten Durchmesser und einem Führungskanal mit einem zweiten Durchmesser, der größer ist als der erste Durchmesser, wobei der erste Durchmesser etwa 3 mm und der zweite Durchmesser etwa 5 mm betragen kann.

In bevorzugter Weiterbildung der Erfindung ist vorgesehen, daß das Rohr mindestens einen zusätzlichen, als Saug-/oder Spülkanal dienenden Kanal aufweist. Vorzugsweise weist das Rohr zwei zusätzliche Kanäle auf, die insbesondere benachbart zu dem Führungskanal mit dem größeren Durchmesser angeordnet sind und, zur optimalen Ausnutzung des zur Verfügung stehenden Querschnitts, einen in etwa dreieckförmigen Querschnitt aufweisen. Die Anordnung der Führungskanäle kann zweckmäßigerweise derart sein, daß der Winkelabstand zwischen den beiden Führungskanälen mit kleineren Durchmesser, bezüglich der Längs-Mittelachse des Rohrs gesehen, 90° und der Winkelabstand zwischen den Führungskanälen mit kleinerem Durchmesser einerseits und dem Führungskanal mit größerem Durchmesser andererseits jeweils 135° beträgt.

Zum Zu- bzw. Ableiten des im zusätzlichen Kanal geführten Fluids in den zusätzlichen Kanal bzw. aus dem zusätzlichen Kanal ist gemäß einem weiteren vorteilhaften Merkmal der Erfindung vorgesehen, daß jeder der zusätzlichen Kanäle am proximalen Endabschnitt des Rohrs einen radial nach außen verlaufenden Abgang aufweist, der mit einem Saug- bzw. Spülanschluß, der am Bedienungshandgriff angebracht ist, in Verbindung steht. Weiterhin ist vorgesehen, daß im Kontaktbereich zwischen dem Rohr und dem das Rohr im Bereich des Abgangs/der Abgänge dichtend umgebenden Bauteil des Bedienungshandgriffs ein einerseits mit dem Abgang und andererseits mit dem Saug- bzw. Spülanschluss in Verbindung stehender, in Umfangsrichtung des Rohrs verlaufender Raum vorgesehen ist, der sicherstellt, daß auch bei Drehung des Rohrs relativ zum Bauteil die Fluidverbindung zwischen dem zusätzlichen Kanal und dem Saug- bzw. Spülanschluß aufrechterhalten bleibt.

Weitere vorteilhafte Merkmale der Erfindung ergeben sich aus den übrigen Unteransprüchen sowie aus der nachfolgenden Beschreibung, in der mehrere Ausführungsbeispiele der Erfindung anhand der Zeichnung näher erläutert werden. In der Zeichnung zeigen in halbschematischer bzw. schematischer Darstellung:
- Fig. 1: eine teilweise geschnittene Seiten-Teilansicht eines erfindungsgemäßen Multifunktionsinstruments,
- Fig. 2: eine teilweise geschnittene Ansicht des Multifunktionsinstruments gemäß Fig. 1,
- Fig. 3: eine Teilansicht eines modifizierten Ausführungsbeispiels eines Multifunktionsinstruments gemäß Fig. 1,
- Fig. 4: eine teilweise geschnittene Teilansicht, die den Antrieb für das Rohr zeigt,
- Fig. 5: eine Teilansicht, die die Funktionsstellung zeigt, bei der ein erstes chirurgisches Instrument verschiebbar ist,
- Fig. 6: eine Teilansicht, die die Funktionsstellung zeigt, bei der ein zweites chirurgisches Instrument drehbar ist,
- Fig. 7: eine Teilansicht, die die Funktionsstellung zeigt, bei der das erste chirurgische Instrument drehbar ist,
- Fig. 8: eine Teilansicht, die die Funktionsstellung zeigt, bei der ein drittes chirurgisches Instrument drehbar ist,
- Fig. 9: eine Teilansicht, die die Funktionsstellung zeigt, bei der das dritte chirurgische Instrument verschiebbar ist, und

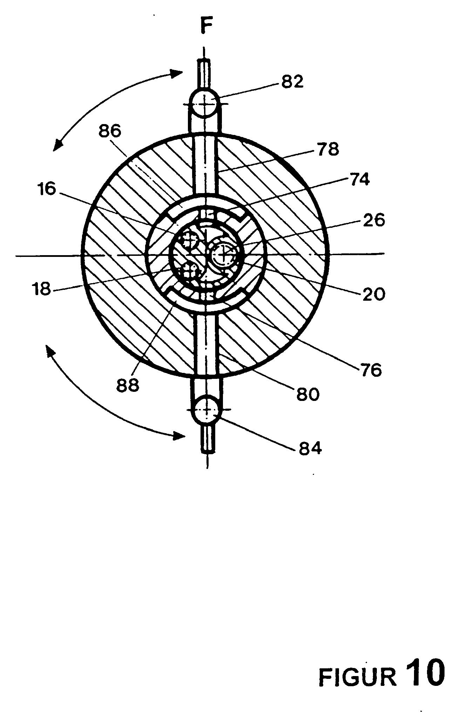

- Fig. 10: einen radialen Schnitt durch das proximale Ende des Rohrs.

Das erfindungsgemäße Multifunktionsinstrument umfaßt einen pistolenförmigen, abgewinkelten Bedienunghandgriff 10 mit einem Gehäuse 12 sowie ein am Bedienungshandgriff 10 abnehmbar und über Lagerschalen oder Buchsenlagerung drehbar gelagertes mehrlumiges Rohr 14 mit drei koaxial zur Rohrachse ausgebildeten Führungskanälen 16, 18, 20, in denen chirurgische Instrumente 22, 24, 26 in Längsrichtung verschieblich und zusätzlich auch drehbar gelagert sind. Jedes der chirurgischen Instrumente 22, 24, 26 weist an seinem hier nicht dargestellten, distalen Ende ein Operationswerkzeug wie beispielsweise eine Schere, einen Fassapplikator oder einen Clipapplikator auf, der jeweils mit einem Schaft 220, 240 bzw. 260 verbunden ist. Jeder Schaft weist an seinem proximalen Endabschnitt einen ersten gezahnten Abschnitt 222, 242, 262 sowie einen zweiten gezahnten Abschnitt 224, 244, 264 auf, der an den ersten gezahnten Abschnitt in Richtung zum distalen Ende hin anschließt. Bei in das Rohr 14 eingesetzten chirurgischen Instrumenten, die sich in Ruhestellung befinden, ragen die Schafte der chirurgischen Instrumente mit ihrem proximalen Endabschnitt aus dem proximalen Ende des Rohrs 14 heraus, so, daß die gezahnten Abschnitte der Schafte selektiv mit Antriebselementen in Eingriff gebracht werden können, derart, daß das jeweils gewünschte der chirurgischen Instrumente 22, 24, 26 aus seiner Ruhestellung, in der das Arbeitswerkzeug in den zugeordneten Führungskanal zurückgezogen ist, in eine Arbeitsposition vorfahren kann, in der das selektierte chirurgische Arbeitswerkzeug aus dem distalen Ende des Rohrs 14 herausragt und in dieser Position dann manuell oder motorisch betätigbar und wenigstens eines der Instrumente auch motorisch drehbar ist.

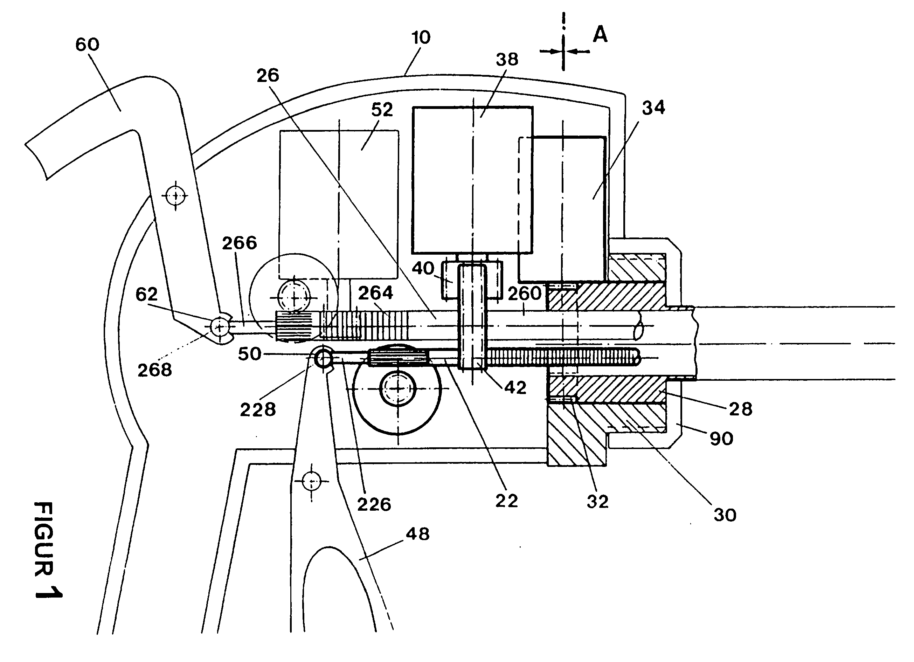

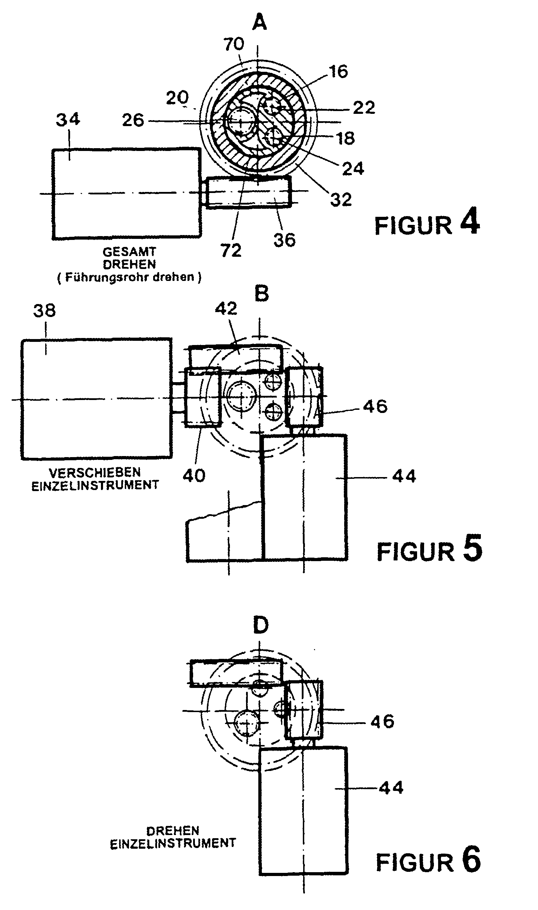

Das Rohr 14 weist an seinem proximalen Ende einen Lagerabschnitt 28 mit gegenüber dem übrigen Rohr vergrößertem Durchmesser auf, der in einem am distalen Ende des ingesamt etwa pistolenförmigen Bedienungshandgriffs 10 vorgesehenen Lagerblock 30 drehbar gelagert ist. Am proximalen Ende des Lagerblocks 30 ist eine über seinen Umfang verlaufende Verzahnung 32 ausgebildet. Ein im Gehäuse 12 des Bedienungshandgriffs 10 angeordneter Elektromotor 34 greift mit einem an seiner Ausgangswelle befestigten Motorritzel 36 in die Verzahnung 32 des Lagerabschnitts 28 des Rohrs 14 antriebsmäßig ein, wodurch das Rohr 14 mittels des Elektromotors 34 gedreht werden kann, vergleiche insbesondere Fig. 1 und 4.

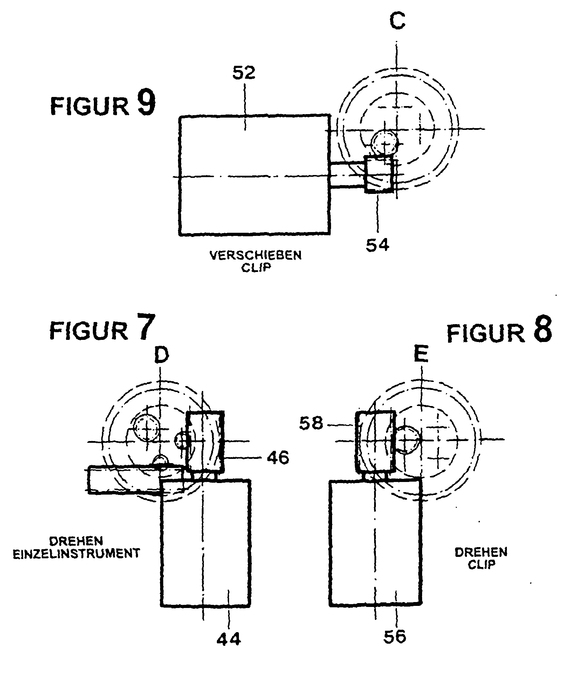

Zum Verschieben der chirurgischen Instrumente 22, 24, bei denen es sich beispielsweise um eine Schere, einen Fassapplikator oder einen Tasthaken handeln kann, ist ein im Gehäuse 12 befestigter Elektromotor 38 vorgesehen, der über ein Motorritzel 40 und ein Zwischenritzel 42 antriebsmäßig mit der Radialverzahnung des zweiten gezahnten Abschnitts 224 bzw. 244 des jeweiligen Schafts 220 bzw. 240 zusammenwirkt, vergleiche insbesondere Figuren 1 und 5. Das Zwischenritzel 42 des Elektromotors 38 kommt mit dem zweiten gezahnten Abschnitt des jeweiligen Schafts dadurch in Eingriff, daß das Rohr 14 mittels des Elektromotors 34 in die entsprechende Winkelstellung gedreht wird. Ausgehend von einer Basisposition des Rohrs bzw. der chirurgischen Instrumente, wie sie in Fig. 5 gezeigt wird, schwenken die chirurgischen Instrumente aufgrund entsprechender Drehung des Rohres entsprechend den Figuren 6 und 7 in die Verzahnung des Zwischenritzels 42.

Über den Motor 38 kann das mit dem Zwischenritzel 42 in Eingriff stehende chirurgische Instrument somit in axialer Richtung verschoben werden. Sobald das selektierte chirurgische Instrument in die Arbeitsposition ausgefahren wurde, wird es, durch Drehen des Rohres 14 um 90°, mit seiner Stirnverzahnung im ersten verzahnten Abschnitt 222 bzw. 242 in Eingriff mit einem Motorritzel 46 gebracht, welches an der Ausgangswelle eines im Gehäuse 12 des Bedienungshandgriffs 10 befestigten Elektromotors 44 befestigt ist. Mittels des Motors 44 kann somit das jeweils selektierte chirurgische Instrument 22 oder 24 beliebig gedreht werden. Am proximalen Ende des Schafts 220, 240 des chirurgischen Instruments 22, 24 ist jeweils eine mit dem Schaft koaxiale Stange 226, 246 ausgebildet, an deren proximalem Ende eine Kupplungskugel 228, 248 angeformt ist. Ein am Gehäuse schwenkbar gelagerter Bedienungs-Schwenkhebel 48, dessen einer Hebelarm in das Gehäuse 12 hineinragt, weist am Ende dieses Hebelarms eine Aufnahmeöffnung 50 auf, deren Form der Form der Kupplungskugel 228, 248 angepasst ist und die die Kupplungskugel 228, 248 einrastbar und lösbar aufnehmen kann. In der in den Figuren 6 und 7 dargestellten Position wird das selektierte chirurgische Instrument 22 oder 24 nach hinten verfahren, bis seine Kupplungskugel 228, 248 in die Aufnahmeöffnung 50 des Bedienungs-Schwenkhebels 48 eintritt und einrastet. Nunmehr ist eine direkte, manuelle Betätigung des Arbeitswerkzeugs möglich, was insbesondere für solche chirurgische Instrumente sinnvoll ist, die eine taktile Rückmeldung erfordern, wie beispielsweise Scheren.

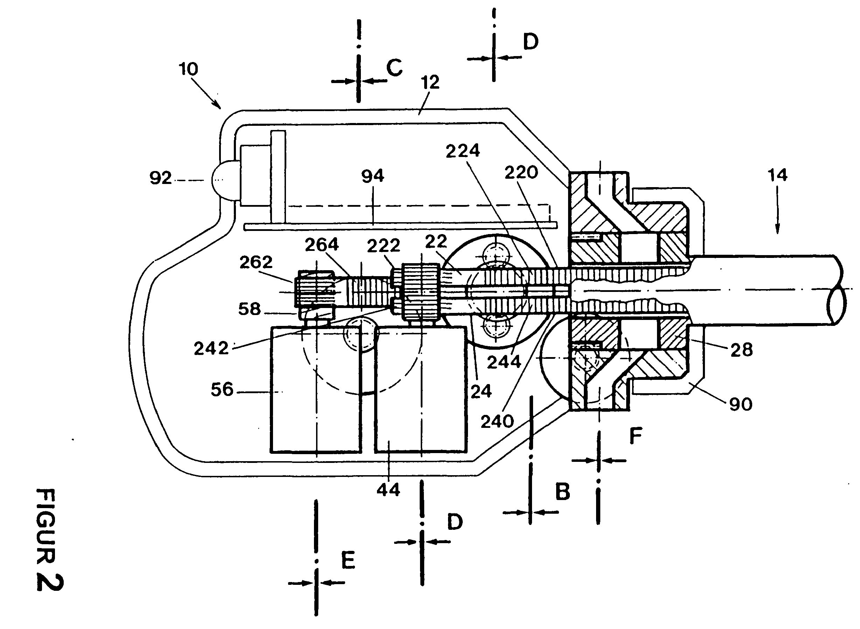

Das chirurgische Instrument 26, welches an seinem distalen Ende beispielsweise mit einem Clipapplikator versehen sein kann, ist mittels eines Elektromotors 52 verschiebbar, der fest im Gehäuse 12 des Bedienungshandgriffs 10 angeordnet ist und über ein an dessen Ausgangswelle befestigtes Motorritzel 54 mit der Radialverzahnung des zweiten gezahnten Abschnitts 264 des Schafts 260 des chirurgischen Instruments 26 bei entsprechender Winkelposition des Rohrs 14 antriebsmäßig in Eingriff bringbar ist, vergleiche Fig. 1 und 9. Ein weiterer, im Gehäuse befestigter Elektromotor 56 steht über ein an dessen Ausgangswelle befestigtes Motorritzel 58 bei entsprechender Position des Rohrs 14 mit der Stirnverzahnung des ersten gezahnten Abschnitts 262 des Schafts 260 des chirurgischen Instruments 26 in antriebsmäßigem Eingriff und ermöglicht somit eine Rotation des chirurgischen Instruments 26, vergleiche Fig. 2 und 8. Das Einschwenken des chirurgischen Instruments 26 in die Antriebsposition gemäß Fig. 8 und 9 erfolgt durch entsprechendes Drehen des Rohres 14, wie weiter oben beschrieben.

Entsprechend den chirurgischen Instrumenten 22 und 24 weist auch das chirurgische Instrument 26 an seinem proximalen Ende eine Stange 266 auf, an deren proximalem Ende eine Kupplungskugel 268 ausgebildet ist, die in eine Aufnahmeöffnung 62 eines weiteren Bedienungs-Schwenkhebels 60 einrastbar ist, der schwenkbar am Gehäuse 12 gelagert ist und an seinem in das Innere des Gehäuses 12 ragenden Schwenkarm die Aufnahmeöffnung 62 aufweist. In der Arbeitsstellung des chirurgischen Instruments 26 ist die Kupplungskugel 268 in der Aufnahmeöffnung 62 eingerastet und das chirurgische Instrument 26 ebenso wie die chirurgischen Instrumente 22 und 24 somit direkt manuell bedienbar.

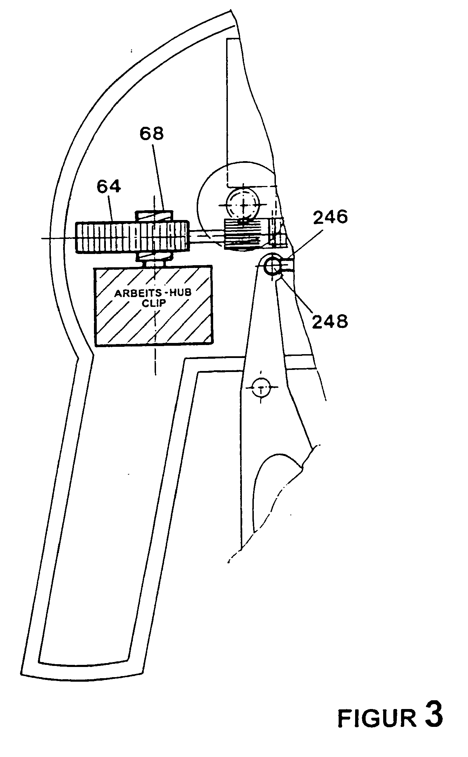

Instrumente, die keine taktile Rückmeldung der Gewebekonstitution benötigen, sondern als Arbeitshub nur vor und zurück fahren müssen, beispielsweise Instrumente zum Setzen eines Clips, können auch motorisch betätigt werden, vergleiche Fig. 3, die insoweit eine alternative Ausführungsform zeigt. Anstelle der Kupplungskugel 268 ist hier eine Zahnstange 64 mit Radial-Verzahnung vorgesehen, die in der Arbeitsposition des chirurgischen Instruments 26 mit einem Motorritzel 68 eines im Gehäuse 12 befestigten Elektromotors 66 in antriebsmäßigem Eingriff steht.

Im Falle des dargestellten Ausführungsbeispiels ist der Durchmesser der die chirurgischen Instrumente 22, 24 aufnehmenden Führungskanäle 16, 18 3 mm, wobei die chirurgischen Instrumente 22, 24 einen etwa gleich großen Arbeitshub aufweisen. Der Durchmesser des das chirurgische Instrument 26 aufnehmenden Führungskanals 20 beträgt im Falle dieses Ausführungsbeispiels 5 mm und dieses chirurgische Instrument kann einen gegenüber den chirurgischen Instrumenten kürzeren oder längeren Arbeitshub aufweisen.

Bezüglich der Mittelachse des Rohrs sind die beiden Führungskanäle 16, 18 in einem Winkelabstand von 90° angeordnet und der Winkelabstand des Führungskanals 20 zu dem Führungskanal 16 und dem Führungskanal 18 beträgt jeweils 135°. Die zwischen dem Führungskanal 16 und dem Führungskanal 20 sowie dem Führungskanal 18 und dem Führungskanal 20 befindlichen Räume sind zur Ausbildung weiterer Kanäle 70, 72 genutzt, die insbesondere als Spül- und Saugkanäle Verwendung finden können. Zur optimalen Raumausnutzung sind die Kanäle 70 im Querschnitt in etwa dreieckförmig ausgebildet, mit gewölbten Seitenflächen entsprechend den benachbarten Wandungen.

Die Kanäle 70, 72 öffnen sich zum distalen Ende des Rohrs hin in axialer Richtung und weisen am proximalen Ende des Rohrs 14 im Bereich des Lagerabschnitts 28 je einen radialen Abgang 74, 76 auf, der auf je einen im Lagerblock 30 ausgebildeten Saug- bzw Spül-Anschlußkanal 78, 80 läuft, auf dem die Saug- bzw. Spül-Anschlüsse 82, 84 aufgesetzt sind. Weiterhin schließt sich an das außenliegende Ende eines jeden radial Abgangs 74, 76 jeweils ein Raum 86, 88 an, der sich in Umfangsrichtung des Rohrs zu beiden Seiten des Abgangs über einen Winkelbereich von insgesamt beispielsweise 90° erstreckt und nach außen hin offen ist. Auf diese Weise bleibt auch bei Drehung des Rohrs 14 relativ zum Bedienungshandgriff 10 die Verbindung zwischen dem jeweiligen radialen Abgang des Kanals und dem zugeordneten Saug-/Spülanschluß erhalten.

Wie bereits erwähnt ist das aus Kunststoff bestehende Rohr 14 am Bedienungshandgriff 10 lösbar und es ist mit seinem Lagerabschnitt 28 in den Lagerblock 30 des Bedienungshandgriffs 10 in axialer Richtung einsteckbar und mittels einer Überwurfmutter 90, die mittels eines Unverlierbarkeitsrings gesichert ist, in seiner Funktionsposition fixiert. Die Abnehmbarkeit des Rohrs 14 vom Bedienungshandgriff 10 ermöglicht einerseits eine einfachere Reinigung des Rohrs 14 und andererseits das einfache Bestücken des Rohrs 14 von hinten.

Die Bedienung des Multifunktionsinstruments erfolgt, soweit sie nicht über die Bedienungs-Schwenkhebel 48 und 60 erfolgt, über eine nicht dargestellte elektronische Steuerung, die außerhalb des Bedienungshandgriffs 10 angeordnet sein kann und mit diesem über ein Kabel verbunden sein kann. Die Steuerungsbefehle erhält die elektronische Steuerung entweder über ein am Gehäuse 12 des Bedienungshandgriffs 10 angeordnetes Tastenfeld, welches in Fig. 2 mit der Bezugsziffer 92 angedeutet ist, oder über eine Spracheingabesteuerung. Insbesondere sind folgende Steuerungsbefehle vorgesehen: Instrument 22 vor und zurück, Instrument 24 vor und zurück, Instrument 26 vor und zurück, in Arbeitsposition befindliches Instrument drehen rechts oder drehen links, in Arbeitsposition befindliches Instrument 26 Arbeitshub (vergleiche Fig. 3).

Im Falle des Ausführungsbeispiels gemäß Fig. 2 befindet sich die eigentliche elektronische Steuerung innerhalb des Gehäuses 12 und ist durch die Leiterplatte 94. angedeutet. Bei allen Ausführungsformen erfassen nicht näher dargestellte Sensoren die Positionen der jeweiligen Instrumente. Diese Positionsdaten werden in der elektronischen Steuerung verarbeitet und entsprechend den über das Tastenfeld oder die Spracheingabe eingegebenen Steuerbefehlen werden dann die entsprechenden Elektromotoren angesteuert. Im Falle einer Sprachsteuerung wird diese auf einem externen Rechner durchgeführt, der dann der im Gehäuse 10 befindlichen elektronischen Steuerung oder unmittelbar den Elektromotoren die entsprechenden Befehle übermitteln kann.

Zur Durchführung einer Koagulation muß das Rohr 14 isoliert sein. Bereits aus diesem Grunde ist das Rohr 14 aus Kunststoff gefertigt. Zur Koagulation wird eines der chirurgischen Instrumente 22, 24 verwendet. Die Zuführung der zur Koagulation erforderlichen Spannung erfolgt durch Kontaktierung an der Mantelfläche des Instruments 22, 24 oder aber auch an der jeweiligen Kupplungskugel 228, 248. Der monopolare Anschluß erfolgt auf das Gehäuse 12 oder auf dem Bedienungs-Schwenkhebel 48.

Das erfindungsgemäße Multifunktionsinstrument erlaubt es somit, innerhalb des Rohres 14 drei unterschiedliche chirurgische Instrumente zu benutzen, ohne daß der Operateur das Rohr 14 hierzu aus dem Trokar ziehen müßte. Die chirurgischen Instrumente werden über geeignete Betätigungselemente am Bedienungshandgriff 10 oder über Sprachsteuerung ausgewählt, in Arbeitsposition gebracht, gegebenenfalls an die Bedienungs-Schwenkhebel zur manuellen Betätigung angekuppelt, oder, falls ein motorischer Arbeitshub erfolgen soll, motorisch betätigt. Die für verschieden Operationsschritte erforderlichen chirurgischen Instrumente werden damit im Operationsfeld simultan und rasch austauschbar vorgehalten.

Das erfindungsgemäße Multifunktionsinstrument ermöglicht die Einsparung von OP-Zeit. Zusätzliche Einsparungspotentiale ergeben sich dadurch, daß für größere Zeiteinheiten der Operation eine Instrumentierschwester nicht erforderlich ist, da keine Instrumente gewechselt werden müßen, solange mit dem erfindungsgemäßen Multifunktionsinstrument alle Instrumentenfunktionen im Operationsfeld, zum Beispiel der Bauchhöhle, zur Verfügung stehen.

Ein weiterer wesentlicher Vorteil des erfindungsgemäßen Multifunktionsinstruments besteht darin, daß es, im Gegensatz zu Multifunktionsintrumenten nach dem Stand der Technik, einfach an einen medizinischen Roboter adaptiert werden kann, nachdem in absehbarer Zeit die unterschiedlichsten Operationen wie Herzkranzgefäß-Operationen oder Eingriffe an Wirbelsäule und Gelenken in zunehmendem Maße nicht unmittelbar vom Operateur selbst, sondern vielmehr unter Zwischenschaltung von Roboterarmen (Telemedizin-Robotics) durchgeführt werden. Ein Multifunktionsinstrument gemäß der vorliegenden Erfindung, zwischengeschaltet zwischen Roboterarm und Operationsgebiet, erhöht die Einsatzmöglichkeit, nachdem hier der einzelne Roboterarm nicht mehr auf ein einziges Instrument festgelegt ist, sondern mit variablen Funktionen ausgestattet werden kann.

Ein weiterer Vorteil des erfindungsgemäßen Multifunktionsinstruments ergibt sich aufgrund des Umstands, daß eine Operationsschwester oder ein Operationsassistent, der die jeweils erforderlichen Instrumente reicht, nicht mehr erforderlich ist und mögliche Kommunikationsfehler, zum Beispiel Anreichung falscher Instrumente, die den Operationsablauf stören oder das operative Ergebnis gefährden könnten, wegfallen.

Die Vorgehensweise mit dem erfindungsgemäßen Multifunktionsgerät ist, kurz zusammengefasst, wie folgt:

Die wiederverwendbaren Instrumente bzw. Instrumententeile vorhergehender Operationen werden sterilisiert. Es wird präoperativ eine Auswahl der gewünschten Einsatzinstrumente wie z. B. Schere, Fasszange, Clip usw. getroffen. Die ausgewählten Instrumente werden in das vom Bedienungshandgriff abgenommene Rohr von hinten in die jeweiligen Führungskanäle eingesetzt. Das Rohr wird auf den Bedienungshandgriff aufgesetzt und mittels der Überwurfmutter 90 gesichert. Die einzelnen Instrumente werden automatisch in ihre Ruheposition innerhalb des Rohrs eingezogen und positioniert. An dem Bedienungshandgriff werden gegebenenfalls externe (unsterile) Hilfsgeräte angekoppelt. Intraoperativ erfolgt über am Gehäuse befindliche Tasten oder über Sprachsteuerung eine Auswahl des chirurgischen Instruments, welches dann automatisch in Arbeitsposition gefahren und am Bedienungs-Schwenkhebel angekoppelt wird. Wird ein Wechsel des Instruments gewünscht und das entsprechende Instrument durch Tastendruck oder Sprachsteuerung angesteuert, so wird das bisher verwendete Instrument automatisch in seine Ruheposition verbracht, das gewählte nächste Instrument wird automatisch ausgewählt und automatisch in Arbeitsstellung gebracht. Das in Arbeitsstellung befindliche Instrument kann jederzeit gedreht werden. Nach Beendigung des operativen Eingriffs und Verbringung des zuletzt benutzten chirurgischen Instruments in seine Ruhelage wird das Rohr aus dem Körper des Patienten entfernt, vom Bedienungshandgriff abgenommen und einschließlich der eingesetzten chirurgischen Instrumente gereinigt.

Für den Fachmann ergeben sich zahlreiche Modifikationsmöglichkeiten. So kann z.B. die Ankoppelung der chirurgischen Instrumente elektrisch, pneumatisch oder hydraulisch mit Energieträger im Handgriff oder extern im unsterilen OP-Bereich erfolgen. Falls die Rotation der chirurgischen Werkzeuge nicht erforderlich ist, kann auf die entsprechenden motorischen Antriebsmittel insoweit verzichtet werden. Die motorische Drehung der Einzelinstrumente kann auch dadurch erfolgen, daß das Rohr ingesamt gedreht wird.

### Bezugszeichenliste

- 10: Bedienungshandgriff
- 12: Gehäuse
- 14: Rohr
- 16: Führungskanal
- 18: Führungskanal
- 20: Führungskanal

- 22: chirurgisches Instrument
- 220: Schaft
- 222: 1. gezahnter Abschnitt
- 224: 2. gezahnter Abschnitt
- 226: Stange
- 228: Kupplungskugel

- 24: chirurgisches Instrument
- 240: Schaft
- 242: 1. gezahnter Abschnitt
- 244: 2. gezahnter Abschnitt
- 246: Stange
- 248: Kupplungskugel

- 26: chirurgisches Instrument
- 260: Schaft
- 262: 1. gezahnter Abschnitt
- 264: 2. gezahnter Abschnitt
- 266: Stange
- 268: Kupplungskugel

- 28: Lagerabschnitt
- 30: Lagerblock
- 32: Verzahnung
- 34: Elektromotor
- 36: Motorritzel
- 38: Elektromotor
- 40: Motorritzel
- 42: Zwischenritzel
- 44: Elektromotor
- 46: Motorritzel
- 48: Bedienungs-Schwenkhebel
- 50: Aufnahmeöffnung
- 52: Elektromotor
- 54: Motorritzel
- 56: Elektromotor
- 58: Motorritzel
- 60: Bedienungs-Schwenkhebel
- 62: Aufnahmeöffnung
- 64: Zahnstange
- 66: Elektromotor
- 68: Motorritzel
- 70: Kanal
- 72: Kanal
- 74: radialer Abgang
- 76: radialer Abgang
- 78: Anschlußkanal
- 80: Anschlußkanal
- 82: Saug-/Spülanschlüsse
- 84: Saug-/Spülanschlüsse
- 86: Raum
- 88: Raum
- 90: Überwurfmutter
- 92: Tastenfeld
- 94: Leiterplatte

## Patentansprüche

1. Multifunktionsinstrument für die mikroinvasive Chirurgie mit einem Bedienungshandgriff (10), einem am Bedienungshandgriff befestigten mehrlumigen Rohr (14), mindestens zwei im Rohr koaxial ausgebildeten Führungskanälen (16, 18, 20) und mit in den Führungskanälen verschieblich und drehbar gelagerten chirurgischen Instrumenten (22, 24, 26) mit einem Schaft (220, 240, 260), an dessen distalem Ende jeweils ein chirurgisches Arbeitselement angeordnet ist, wobei die Instrumente (22, 24, 26) zwischen einer Ruheposition, in der das jeweilige Arbeitselement in das Rohr zurückgezogen ist, und einer Arbeitsposition, in der das jeweilige Arbeitselement an dem distalen Ende des Rohrs herausragt, verschieblich ist,
**dadurch gekennzeichnet, daß**
die Instrumente (22, 24, 26) in die und aus der Arbeitsposition motorisch verfahrbar sind,
wenigstens eines der Instrumente (22, 24, 26) in seiner Arbeitsposition motorisch drehbar ist, und
eine elektronische Steuerung zum Steuern zumindest der motorischen Verfahrbewegungen und Drehbewegungen vorgesehen ist.

2. Multifunktionsinstrument nach Anspruch 1, **dadurch gekennzeichnet, daß** das mehrlumige Rohr (14) am Bedienungshandgriff (10) drehbar gelagert ist und motorisch drehbar ist, wobei die Drehbewegung mittels der elektronischen Steuerung steuerbar ist.

3. Bedienungshandgriff nach Anspruch 1 oder 2, **dadurch gekennzeichnet, daß** zum motorischen Antrieb innerhalb des Bedienungshandgriffs (10) Elektromotoren (34, 38, 44, 52, 56, 66) angeordnet sind.

4. Multifunktions instrument nach Anspruch 3, **dadurch gekennzeichnet, daß** die den Instrumenten zugeordneten Elektromotoren über zwischen das jeweilige Instrument und dem (den) zugeordneten Elektromotor(en) geschaltete Kopplungselemente (40, 42, 46, 54, 58, 68) selektiv mit dem jeweils auszuwählenden Instrument (22, 24, 26) koppelbar sind.

5. Multifunktionsinstrument nach Anspruch 4, **dadurch gekennzeichnet, daß** die selektive Kopplung durch eine Relativbewegung zwischen Instrument und zugeordnetem/zugeordneten Elektromotor(en) bzw. dem (den) zugeordneten Kopplungselement(en) erfolgt.

6. Multifunktionsinstrument nach Anspruch 5, **dadurch gekennzeichnet, daß** die Elektromotoren im Bedienungshandgriff (10) ortsfest angeordnet sind und die selektive Kopplung im wesentlichen durch Rotation des Rohrs (14) erfolgt.

7. Multifunktionsinstrument nach einem der vorhergehenden Ansprüche, **dadurch gekennzeichnet, daß** ein proximaler Endabschnitt des Schafts (220, 240, 260) eines jeden Instruments (22, 24, 26), der im in das Rohr (14) eingesetzten Zustand aus dem proximalen Ende des Rohrs heraussteht, Zahnungen (222, 242, 262; 224, 244, 264) zum Eingriff mit dem motorischen Antrieb aufweist.

8. Multifunktionsinstrument nach Anspruch 7, **dadurch gekennzeichnet, daß** der Schaft (220, 240, 260) zumindest eines der Instrumente (16, 18, 20) einen ersten Zahnabschnitt (222, 242, 262) für das Verschieben des Instruments in axialer Richtung und einen zweiten Zahnabschnitt (224, 244, 264) für das Drehen des Instruments aufweist.

9. Multifunktionsinstrument nach Anspruch 7 oder 8, **dadurch gekennzeichnet, daß** der Schaft (220, 240, 260) zumindest eines der Instrumente (22, 24, 26) an seinem proximalen Ende ein Kupplungselement (228, 248, 268) zum Eingriff mit einem Betätigungselement (48, 60) zum manuellen Betätigen des Instruments aufweist.

10. Multifunktioninstrument nach einem der vorhergehenden Ansprüche, **dadurch gekennzeichnet, daß** das Rohr (14) Führungskanäle (16, 18, 20) unterschiedlichen Durchmessers aufweist.

11. Multifunktionsinstrument nach einem der vorhergehenden Ansprüche, **dadurch gekennzeichnet, daß** das Rohr (14) mindestens drei Führungskanäle (16, 18, 20) aufweist.

12. Multifunktionsinstrument nach Ansprüchen 10 und 11, **dadurch gekennzeichnet, daß** das Rohr (14) zwei Führungskanäle (16, 18) mit einem ersten Durchmesser und einen Führungskanal (20) mit einem zweiten Durchmesser aufweist, der größer als der erste Durchmesser ist.

13. Multifunktionsinstrument nach Anspruch 12, **dadurch gekennzeichnet, daß** der erste Durchmesser 3 mm +/-0,5 mm und der zweite Durchmesser 5 mm +/- 0,5 mm beträgt.

14. Multifunktionsinstrument nach Anspruch 12 oder 13, **dadurch gekennzeichnet, daß** der Winkelabstand zwischen den beiden Führungskanälen (16, 18) mit kleinem Durchmesser, bezüglich der Längs-Mittelachse des Rohrs (14) gesehen, 90° und zwischen den Führungskanälen (16, 18) mit kleinem Durchmesser und dem Führungskanal (18) mit größerem Durchmesser jeweils 135° beträgt.

15. Multifunktionsinstrument nach einem der vorhergehenden Ansprüche, **dadurch gekennzeichnet, daß** das Rohr (14) mindestens einen zusätzlichen, als Saug-/oder Spülkanal dienenden Kanal (70, 72) aufweist.

16. Multifunktionsinstrument nach Anspruch 15, **dadurch gekennzeichnet, daß** das Rohr (14) zwei zusätzliche Kanäle (70, 72) aufweist.

17. Multifunktionsinstrument nach einem der Ansprüche 12 bis 14 und nach Anspruch 16, **dadurch gekennzeichnet, daß** die beiden zusätzlichen Kanäle (70, 72) benachbart zu dem Führungskanal (18) mit dem größeren Durchmesser ausgebildet sind.

18. Multifunktionsinstrument nach Anspruch 17, **dadurch gekennzeichnet, daß** die beiden zusätzlichen Kanäle (70, 72) jeweils einen in etwa dreieckförmigen Querschnitt aufweisen.

19. Multifunktionsinstrument nach einem der Ansprüche 15 bis 18, **dadurch gekennzeichnet, daß** jeder der zusätzlichen Kanäle (70, 72) am proximalen Endabschnitt des Rohrs (14) einen radial nach außen verlaufenden Abgang (74, 76) aufweist, der mit einem Saug- bzw. Spülanschluß (82, 84) in Verbindung steht.

20. Multifunktionsinstrument nach Anspruch 19, **dadurch gekennzeichnet, daß** im Kontaktbereich zwischen dem Rohr (14) und dem das Rohr im Bereich des Abgangs/der Abgänge dichtend umgebenden Bauteil (30) des Bedienungshandgriffs (10) ein einerseits mit dem Abgang (74, 76) und andererseits mit dem Saug- bzw. Spülanschluß (82, 84) in Verbindung stehender, in Umfangsrichtung des Rohrs verlaufender Raum (86, 88) vorgesehen ist, der derart ausgebildet ist, daß auch bei Drehung des Rohrs (14) relativ zum Bauteil (30) die Fluidverbindung zwischen dem zusätzlichen Kanal und dem Saug- bzw. Spülanschluß aufrechterhalten bleibt.

21. Multifunktionsinstrument nach einem der vorhergehenden Ansprüche, **dadurch gekennzeichnet, daß** das Rohr (14) mit dem Bedienungshandgriff (10) zum Zwecke des Beladens mit und Entladens von Instrumenten (22, 24, 26) lösbar verbunden ist, insbesondere über eine Steckverbindung (28, 30) mit Überwurfmutter (90).

22. Multifunktionsinstrument nach einem der vorhergehenden Ansprüche, **dadurch gekennzeichnet, daß** mindestens zwei der chirurgischen Instrumente (22, 24) einen kurzen Arbeitshub bei taktiler Rückmeldung aufweisen, z.B. Schere und Fassapplikator, und, nach motorischem Verbringen in ihre Arbeitsposition, manuell betätigbar sind, und daß mindestens eines der chirurgischen Instrumente (26) mit langem Arbeitshub, z.B. Clipapplikator, nach motorischem Verbringen in seine Arbeitsposition manuell oder motorisch betätigbar ist.

23. Multifunktionsinstrument nach einem der vorhergehenden Ansprüche, **dadurch gekennzeichnet, daß** die Befehlseingabe für die Steuerung über am Bedienungshandgriff (10) angeordnete Betätigungselemente (92) erfolgt.

24. Multifunktionsinstrument nach einem der vorhergehenden Ansprüche, **dadurch gekennzeichnet, daß** die Steuerung eine sprachliche Befehlseingabe ermöglicht.

25. Multifunktioninstrument nach Anspruch 23 oder 24, **dadurch gekennzeichnet, daß** die von der Steuerung zu verarbeitenden Befehle zumindest das Einnehmen der Ruhe- oder Arbeitspositionen der Instrumente (22, 24, 26) und/oder das Drehen der Instrumente und/oder das Drehen des Rohrs umfaßt.

26. Multifunktionsinstrument nach Anspruch 23 und 24 oder Anspruch 26, **dadurch gekennzeichnet, daß** die Befehlseingabe wahlweise über Sprache oder über Betätigungselemente (92) erfolgt.

## Claims

1. Multi-functional instrument for micro-invasive surgery having an operating handle (10), a multi-lumen tube (14) attached to the operating handle, at least two guide channels (16, 18, 20) formed co-axially in the tube, and having surgical instruments (22, 24, 26) which are mounted in a displaceable and rotatable manner in the guide channels and have a shaft (220, 240, 260) at whose distal end a surgical working element is disposed in each case, wherein the instruments (22, 24, 26) are displaceable between a rest position in which the respective working element is retracted into the tube and a working position in which the respective working element protrudes from the distal end of the tube, **characterised in that**
the instruments (22, 24, 26) can be displaced into and out of the working position by means of a motor,
at least one of the instruments (22, 24, 26) can be rotated in its working position by means of a motor, and
an electronic control is provided for controlling at least the motorised displacement movements and rotational movements.

2. Multi-functional instrument as claimed in Claim 1, **characterised in that** the multi-lumen tube (14) is mounted in a rotatable manner on the operating handle (10) and can be rotated by means of a motor, wherein the rotational movement can be controlled by means of the electronic control.

3. Operating handle as claimed in Claim 1 or 2, **characterised in that** electric motors (34, 38, 44, 52, 56, 66) are disposed within the operating handle (10) for the purpose of motorised drive.

4. Multi-functional instrument as claimed in Claim 3, **characterised in that** the electric motors allocated to the instruments can be selectively coupled to the instrument (22, 24, 26), to be selected in each case, via coupling elements (40, 42, 46, 54, 58, 68) connected between the respective instrument and the allocated electric motor(s).

5. Multi-functional instrument as claimed in Claim 4, **characterised in that** the selective coupling is effected by a relative movement between the instrument and the allocated electric motor(s) or allocated coupling element(s).

6. Multi-functional instrument as claimed in Claim 5, **characterised in that** the electric motors are disposed in a positionally-fixed manner in the operating handle (10) and the selective coupling is substantially effected by rotating the tube (14).

7. Multi-functional instrument as claimed in any one of the preceding Claims, **characterised in that** a proximal end portion of the shaft (220, 240, 260) of each instrument (22, 24, 26) which protrudes from the proximal end of the tube in the condition in which it is inserted in the tube (14) comprises teeth (222, 242, 262; 224, 244, 264) for engagement with the motorised drive.

8. Multi-functional instrument as claimed in Claim 7, **characterised in that** the shaft (220, 240, 260) of at least one of the instruments (16, 18, 20) comprises a first toothed portion (222, 242, 262) for the displacement of the instrument in the axial direction and a second toothed portion (224, 244, 264) for the rotation of the instrument.

9. Multi-functional instrument as claimed in Claim 7 or 8, **characterised in that** the shaft (220, 240, 260) of at least one of the instruments (22, 24, 26) comprises at its proximal end a coupling element (228, 248, 268) for engagement with an actuating element (48, 60) for the manual actuation of the instrument.

10. Multi-functional instrument as claimed in any one of the preceding Claims, **characterised in that** the tube (14) comprises guide channels (16, 18, 20) having different diameters.

11. Multi-functional instrument as claimed in any one of the preceding Claims,
**characterised in that** the tube (14) comprises at least three guide channels (16, 18, 20).

12. Multi-functional instrument as claimed in Claims 10 and 11, **characterised in that** the tube (14) comprises two guide channels (16, 18) having a first diameter and one guide channel (20) having a second diameter which is larger than the first diameter.

13. Multi-functional instrument as claimed in Claim 12, **characterised in that** the first diameter is 3 mm ± 0.5 mm and the second diameter is 5 mm ± 0.5 mm.

14. Multi-functional instrument as claimed in Claim 12 or 13, **characterised in that** the angular distance between the two guide channels (16, 18) having the small diameter is 90° with respect to the longitudinal central axis of the tube (14) and the angular distance between the guide channels (16, 18) having the small diameter and the guide channel (18) having the larger diameter is 135° in each case.

15. Multi-functional instrument as claimed in any one of the preceding Claims, **characterised in that** the tube (14) comprises at least one additional channel (70, 72) which is used as a suction channel or a flushing channel.

16. Multi-functional instrument as claimed in Claim 15, **characterised in that** the tube (14) comprises two additional channels (70, 72).

17. Multi-functional instrument as claimed in any one of Claims 12 to 14 and as claimed in Claim 16, **characterised in that** the two additional channels (70, 72) are formed adjacent to the guide channel (18) having the larger diameter.

18. Multi-functional instrument as claimed in Claim 17, **characterised in that** the two additional channels (70, 72) each have a cross-section which is approximately triangular in shape.

19. Multi-functional instrument as claimed in any one of Claims 15 to 18, **characterised in that** each of the additional channels (70, 72) comprise at the proximal end portion of the tube (14) an outlet (74, 76) which extends radially outwards and is connected to a suction or flushing connection (82, 84).

20. Multi-functional instrument as claimed in Claim 19, **characterised in that** in the contact region between the tube (14) and the component (30) of the operating handle (10) which surrounds the tube in the region of the outlet(s) in a sealed manner, a space (86, 88) is provided which is connected on the one hand to the outlet (74, 76) and on the other hand to the suction or flushing connection (82, 84), extends in the peripheral direction of the tube and is formed such that the fluidic connection between the additional channel and the suction or flushing connection is maintained even when the tube (14) is rotated relative to the component (30).

21. Multi-functional instrument as claimed in any one of the preceding Claims, **characterised in that** the tube (14) is connected to the operating handle (10) in a releasable manner for the purposes of loading and unloading instruments (22, 24, 26), in particular via a plug-in connection (28, 30) having a coupling ring (90).

22. Multi-functional instrument as claimed in any one of the preceding Claims, **characterised in that** at least two of the surgical instruments (22, 24) have a short working stroke in the case of tactile feedback, e.g., scissors and fixation applicator, and can be manually actuated after they have been brought into their working position by means of a motor, and **in that** at least one of the surgical instruments (26) having a long working stroke, e.g., clip applicator can be manually actuated or actuated by means of a motor after it has been brought into its working position by means of a motor.

23. Multi-functional instrument as claimed in any one of the preceding Claims, **characterised in that** the instructions for the control are input via actuating elements (92) disposed on the operating handle (10).

24. Multi-functional instrument as claimed in any one of the preceding Claims, **characterised in that** the control allows for instructions to be input vocally.

25. Multi-functional instrument as claimed in Claim 23 or 24, **characterised in that** the instructions to be processed by the control at least include the taking up of the rest/working positions of the instruments (22, 24, 26) and/or the rotation of the instruments and/or the rotation of the tube.

26. Multi-functional instrument as claimed in Claim 23 and 24 or Claim 26, **characterised in that** the instructions are selectively input vocally or via actuating elements (92).

## Revendications

1. Instrument multifonctions pour chirurgie microinvasive, comprenant une poignée de manipulation (10), un tube (14) à plusieurs lumières fixé à la poignée de manipulation, au moins deux conduits de guidage (16, 18, 20) aménagés coaxialement dans le tube, et comprenant des instruments chirurgicaux (22, 24, 26) déplaçables en translation et en rotation dans les conduits de guidage et dotés d'une tige (220, 240, 260) à l'extrémité distale de laquelle est disposé chaque fois un élément de travail chirurgical, les instruments (22, 24, 26) pouvant être déplacés en translation entre une position de repos, dans laquelle l'élément de travail respectif est rentré dans le tube, et une position de travail dans laquelle l'élément de travail concerné dépasse de l'extrémité distale du tube, **caractérisé par le fait que**
les instruments (22, 24, 26) peuvent être déplacés par un moteur dans la position de travail et en être retirés,
au moins un des instruments (22, 24, 26) peut être entraîné en rotation par un moteur lorsqu'il est en position de travail, et
une commande électronique est prévue pour commander au moins les translations et les rotations effectuées à l'aide du moteur.

2. Instrument multifonctions selon la revendication 1, **caractérisé par le fait que** le tube (14) à plusieurs lumières est monté tournant sur la poignée de manipulation (10) et peut être entraîné en rotation par un moteur, le mouvement de rotation pouvant être contrôlé au moyen de la commande électronique.

3. Instrument multifonctions selon la revendication 1 ou 2, **caractérisé par le fait que** des moteurs électriques (34, 38, 44, 52, 56, 66) sont disposés à l'intérieur de la poignée de manipulation (10), en vue de l'entraînement par moteur.

4. Instrument multifonctions selon la revendication 3, **caractérisé par le fait que** les moteurs électriques associés aux instruments peuvent être couplés de manière sélective à l'instrument (22, 24, 26) à sélectionner, par l'intermédiaire d'éléments de couplage (40, 42, 46, 54, 58, 68) connectés entre l'instrument concerné et le(s) moteur(s) électrique(s) associé(s).

5. Instrument multifonctions selon la revendication 4, **caractérisé par le fait que** le couplage sélectif s'effectue par un mouvement relatif entre l'instrument et le(s) moteur(s) électrique(s) associé(s) ou l'élément/les éléments de couplage associé(s).

6. Instrument multifonctions selon la revendication 5, **caractérisé par le fait que** les moteurs électriques sont montés stationnaires dans la poignée de manipulation (10) et le couplage sélectif s'effectue essentiellement par rotation du tube (14).

7. Instrument multifonctions selon une des revendications précédentes, **caractérisé par le fait qu'**une portion d'extrémité proximale de la tige (220, 240, 260) de chaque instrument (22, 24, 26) qui, à l'état monté dans le tube (14), dépasse de l'extrémité proximale du tube, présente des dentures (222, 242, 262; 224, 244, 264) destinées à entrer en prise avec le système d'entraînement à moteur.

8. Instrument multifonctions selon la revendication 7, **caractérisé par le fait que** la tige (220, 240, 260) d'au moins un des instruments (16, 18, 20) présente une première portion dentée (222, 242, 262), pour le déplacement de l'instrument dans la direction axiale, et une deuxième portion dentée (224, 244, 264) pour la mise en rotation de l'instrument.

9. Instrument multifonctions selon la revendication 7 ou 8, **caractérisé par le fait que** la tige (220, 240, 260) d'au moins un des instruments (22, 24, 26) présente, à son extrémité proximale, un élément de couplage (228, 248, 268) destiné à venir en prise avec un élément d'actionnement (48, 60), en vue de l'actionnement manuel de l'instrument.

10. Instrument multifonctions selon une des revendications précédentes, **caractérisé par le fait que** le tube (14) présente des conduits de guidage (16, 18, 20) de diamètres différents.

11. Instrument multifonctions selon une des revendications précédentes, **caractérisé par le fait que** le tube (14) présente au moins trois conduits de guidage (16, 18, 20).

12. Instrument multifonctions selon les revendications 10 et 11, **caractérisé par le fait que** le tube (14) comporte deux conduits de guidage (16, 18) avec un premier diamètre et un conduit de guidage (20) avec un deuxième diamètre qui est supérieur au premier diamètre.

13. Instrument multifonctions selon la revendication 12, **caractérisé par le fait que** le premier diamètre est de 3 mm +/- 0,5 mm et le deuxième diamètre est de 5 mm +/- 0,5 mm.

14. Instrument multifonctions selon la revendication 12 ou 13, **caractérisé par le fait que** l'écart angulaire entre les deux conduits de guidage (16, 18) de petit diamètre, rapporté à l'axe longitudinal médian du tube (14), est de 90° et l'écart entre les conduits de guidage (16, 18) de petit diamètre et le conduit de guidage (20) de diamètre plus grand est chaque fois de 135°.

15. Instrument multifonctions selon une des revendications précédentes, **caractérisé par le fait que** le tube (14) présente au moins un conduit (70, 72) supplémentaire, servant de conduit d'aspiration ou de lavage.

16. Instrument multifonctions selon la revendication 15, **caractérisé par le fait que** le tube (14) présente deux conduits (70, 72) supplémentaires.

17. Instrument multifonctions selon une des revendications 12 à 14 et selon la revendication 16, **caractérisé par le fait que** les deux conduits (70, 72) supplémentaires sont aménagés dans le voisinage du conduit de guidage (18) de plus grand diamètre.

18. Instrument multifonctions selon la revendication 17, **caractérisé par le fait que** les deux conduits (70, 72) supplémentaires présentent chacun une section transversale triangulaire.

19. Instrument multifonctions selon une des revendications 15 à 18, **caractérisé par le fait que** chacun des conduits (70, 72) supplémentaires présente, à l'extrémité proximale du tube (14), une sortie (74, 76) qui s'étend radialement vers l'extérieur et communique avec un raccord d'aspiration ou de lavage (82, 84).

20. Instrument multifonctions selon la revendication 19, **caractérisé par le fait que** dans la zone de contact entre le tube (14) et l'élément de construction (30) de la poignée de manipulation (10) qui entoure le tube de façon étanche dans la région de la sortie/des sorties, il est prévu un espace (86, 88), s'étendant dans la direction périphérique du tube, qui communique d'une part avec la sortie (74, 76) et d'autre part avec le raccord d'aspiration ou de lavage (82, 84) et qui est configuré de manière telle que même lors d'une rotation du tube (14) par rapport à l'élément de construction (30), la communication de fluide entre le conduit supplémentaire et le raccord d'aspiration ou de lavage soit conservée.

21. Instrument multifonctions selon une des revendications précédentes, **caractérisé par le fait que** le tube (14) est lié de façon amovible à la poignée de manipulation (10), en particulier par un raccord enfichable (28, 30) comportant un écrou chapeau (90), à des fins de chargement et de déchargement d'instruments (22, 24, 26).

22. Instrument multifonctions selon une des revendications précédentes, **caractérisé par le fait qu'**au moins deux des instruments chirurgicaux (22, 24) présentent une course de travail de faible longueur en cas de réponse tactile, par exemple de ciseaux et d'un élément de préhension, et, après leur déplacement par moteur dans leur position de travail, peuvent être actionnés manuellement, et **par le fait qu'**au moins un des instruments chirurgicaux (26) à course de travail de grande longueur, par exemple un élément à clip, peut être actionné manuellement ou par moteur, après avoir été amené par moteur dans sa position de travail.

23. Instrument multifonctions selon une des revendications précédentes, **caractérisé par le fait que** l'entrée d'instructions pour la commande s'effectue via des éléments d'actionnement (92) disposés sur la poignée de manipulation (10).

24. Instrument multifonctions selon une des revendications précédentes, **caractérisé par le fait que** la commande permet d'effectuer une entrée d'instructions par voie vocale.

25. Instrument multifonctions selon la revendication 23 ou 24, **caractérisé par le fait que** les instructions à traiter par la commande englobent au moins l'occupation des positions de repos ou de travail des instruments (22, 24, 26) et/ou la rotation des instruments et/ou la rotation du tube.

26. Instrument multifonctions selon la revendication 23 ou 24, **caractérisé par le fait que** l'entrée d'instructions s'effectue au choix par voie vocale ou par des éléments d'actionnement (92).
